# EUROPÄISCHE PATENTSCHRIFT

(11) **EP 0 921 771 B1**
(45) Veröffentlichungstag und Bekanntmachung des Hinweises auf die Patenterteilung: **30.10.2002**
(21) Anmeldenummer: 97929294.3
(22) Anmeldetag: 26.06.1997
(51) Int. Cl.: A61F 2/34, G01M 19/00

(54) **VERFAHREN ZUM PRÜFEN VON KERAMISCHEN PFANNEN FÜR HÜFTGELENKENDOPROTHESEN**
METHOD OF TESTING CERAMIC COTYLOID CAVITIES FOR HIP JOINT ENDOPROSTHESES
PROCEDE DE VERIFICATION DE CAVITES COTYLOIDES CERAMIQUES POUR ENDOPROTHESES CORRESPONDANTES

(30) Priorität: 06.07.1996 DE 19627356; 19.12.1996 DE 19652997; 02.05.1997 DE 19718615
(43) Veröffentlichungstag der Anmeldung: 16.06.1999
(73) Patentinhaber: CERASIV GmbH INNOVATIVES KERAMIK-ENGINEERING, D-73207 Plochingen (DE)
(72) Erfinder: AUTENRIETH, Ralph, D-73732 Esslingen (DE); PFAFF, Hans-Georg, D-73760 Ostfildern (DE); RICHTER, Herbert, D-73257 Köngen (DE); WILLMANN, Gerd, D-70771 Leinfelden-Echterdingen (DE); WIMMER, Martin, D-70736 Fellbach (DE); WÖRNE, Christian, D-13353 Berlin (DE)
(74) Vertreter: Scherzberg, Andreas, Dr.
(86) Internationale Anmeldenummer: EP9703351
(87) Internationale Veröffentlichungsnummer: WO98001090

(56) Entgegenhaltungen:
- DE-A- 3 123 437
- DE-A- 4 019 701
- DE-B- 3 001 521
- GB-A- 1 322 680

## Beschreibung

Die Erfindung betrifft ein Verfahren zum Prüfen von keramischen Pfannen für Hüftgelenkendoprothesen nach dem Oberbegriff des Anspruchs 1.

Für künstliche Hüftgelenke werden heute normalerweise modular aufgebaute Systeme verwendet, d.h. auf einem metallischen Schaft mit einem Zapfen wird ein Kugelkopf aufgesetzt, der im allgemeinen gegen eine Pfanne aus Polyethylen artikuliert. Der unvermeidliche Abrieb, insbesondere der des Polyethylens verursacht Osteolyse, die zu einer Lockerung der Prothese und damit zu einer Revisionsoperation führt.

Die Situation kann wesentlich verbessert werden, wenn man anstelle der metallischen Kugelköpfe keramische Kugelköpfe verwendet. Sie wird bestens gelöst, wenn man Kugelköpfe aus Aluminiumoxidkeramik gegen Pfanneneinsätze aus Aluminiumoxidkeramik artikulieren läßt.

Komponenten von Implantaten, die beim Menschen eingesetzt werden sollen, müssen besonders hohen Sicherheitsanforderungen genügen. Dies gilt auch für keramische Komponenten. Da keramische Werkstoffe in allgemeinen spröde sind, können spannungskonzentrierende Inhomogenitäten im Material besonders leicht zu Bruch führen. Dies bedeutet, daß alle Teile, die Defekte enthalten, die unter Einsatzbedingungen zum Versagen führen können, vor dem Einsatz erkannt und ausgesondert werden müssen.

In der bisherigen Qualitätskontrolle können nur Bauteile mit Fehlern an der Oberfläche durch Sichtprüfung ausgesondert werden. Die bekannten Verfahren der zerstörungsfreien Prüfung, wie Röntgendurchleuchtung oder Ultraschallprüfung, sind zwar prinzipiell auch bei keramischen Werkstoffen einsetzbar, sind aber nicht in der Lage, die Defekte mit derjenigen geringen Ausdehnung zu detektieren, die für die geforderte Sicherheit relevant ist.

Oberstes Ziel einer Endkontrolle muß es aber sein, mögliche fehlerhafte Teile sicher auszuschließen, um ein eventuelles Risiko für den Patienten zu minimieren.

Voraussetzung für die Anwendbarkeit und Relevanz eines Proof-Tests ist, daß die Belastung im Proof-Test im Bauteil die Spannungsverteilung, wie sie im Einsatz auftritt in ihrer Verteilung möglichst gut kopiert, und ferner, daß bei Durchführung des Proof-Tests keine Beschädigung des Prüflings durch die Handhabung auftritt.

So ist z. B. ein Proof-Test, bei dem der zu prüfende Pfanneneinsatz in ein reales Metal-back eingesetzt wird, nicht möglich, da nach der Prüfung die Entfernung des Einsatzes aus dem Metal-back nicht ohne Beschädigung möglich ist.

Denkbar wäre eine Prüfung, bei der der keramische Kugelkopf mit einer Überlast (Proof-Test) in die keramische Pfanne bzw. den keramischen Pfanneneinsatz eingedrückt wird. Diese Prüfung ist aber nicht aussagefähig, da hierbei nur eine punktförmige Berührung zwischen Kugelkopf und keramischem Pfanneneinsatz entsteht, also nicht alle Kontaktbereiche zwischen Kugelkopf und Pfanne in der Prüfung nachgestellt werden können.

Der Erfindung liegt die Aufgabe zugrunde, ein Verfahren zum Prüfen von keramischen Pfanneneinsätzen zur Verfügung zu stellen, mit dem sichergestellt ist, daß fehlerhafte Pfanneneinsätze in der Qualitätskontrolle entdeckt und aussortiert werden, ohne daß die Gefahr einer Beschädigung der Pfanneneinsätze besteht.

Erfindungsgemäß wird diese Aufgabe dadurch gelöst,
- daß dabei alle im physiologischen Lastfall unter Last stehenden Volumenelemente der Pfanne belastet werden,
- daß dabei in der Pfanne bzw. im Pfanneneinsatz Spannungen erzeugt werden, die um einen definierten Faktor höher sind, als die im physiologischen Lastfall erzeugten Spannungen.

Die Erfindung betrifft daher eine Proof-Test-Vorrichtung bzw. Verfahren, mit der keramische Pfanneneinsätze der nötigen definierten Überlast ausgesetzt werden können und bei dem die Pfannen aus der Vorrichtung ohne Beschädigung entnommen werden können.

Vorteil dieses Verfahrens ist, daß alle keramischen Pfannen, bei denen kritische Fehler im Volumen bzw. auf der Oberfläche vorhanden sind, unter Last versagen. Dieses Verfahren ist also nicht nur eine Prüfung, die fehlerhafte Bauteile aufzeigt, sondern bei der alle fehlerhaften Teile auch zerstört werden.

Aufgrund von Ergebnissen zur Berechnung der Spannung mit Hilfe der finiten Elementemethode ist die Spannungsverteilung in keramischen Pfannen bekannt.

Die keramischen Pfannen bzw. Pfanneneinsätze bestehen bevorzugt aus hochfester, biokompatibler Keramik, sogenannter Biokeramik. Im speziellen sind dies Aluminiumoxidkeramik (medical-grade aluminia), Zirkonoxidkeramik vom Typ Y-TZP, Werkstoffe auf der Basis Zirkonoxid / Aluminiumoxid, Nichtoxid-Keramiken wie Siliziumnitrid, Siliziumkarbid und Siliziumaluminiumnitrid.

Eine bevorzugte Variante zeichnet sich dadurch aus, daß eine Halbkugel aus einem verformbaren Material unter Last in die Pfanne gedrückt wird.

Vorteilhafterweise ist das verformbare Material der Halbkugel ein Polymer oder ein Kunststoff.

Bei einer alternativen besonders bevorzugten Variante wird die Innenseite der Pfanne mit einer unter Druck stehenden Flüssigkeit belastet. Hiermit können Bruchbilder erzeugt werden, die mit denen aus Berstversuchen mit Hüftgelenkkugeln übereinstimmen. Es kann somit davon ausgegangen werden, daß die Belastung durch Flüssigkeitsdruck die Belastung im Berstversuch gut simuliert.

Vorteilhafterweise wird ein Prüfstempel auf die Pfanne aufgesetzt, der das Innere der Pfanne nach außen mit einem Dichtungselement abdichtet und eine Zuführleitung für die Flüssigkeit aufweist.

Zur Verringerung des notwendigen Flüssigkeitsvolumens ragt der Prüfstempel zweckmäßigerweise mit einer Ausbuchtung in die Pfanne und läßt nur einen Spalt für die Flüssigkeit frei.

Sinnvollerweise wird die zu prüfende Pfanne in eine Haltevorrichtung eingesetzt, wobei erfindungsgemäß die Haltevorrichtung die Pfanne nur an ihrem konisch geformten oberen Ende mittels eines Dichtungselements abstützt. Mit diesem konischen Teil wird die Pfanne in der Metallschale der Hüftgelenkendoprothese verankert.

Erfindungsgemäß ist die Flüssigkeit eine für Hochdruck geeignete Flüssigkeit, z. B. Wasser oder Glycerin.

Weitere Merkmale der Erfindung ergeben sich aus den Figuren, die nachfolgend beschrieben sind.

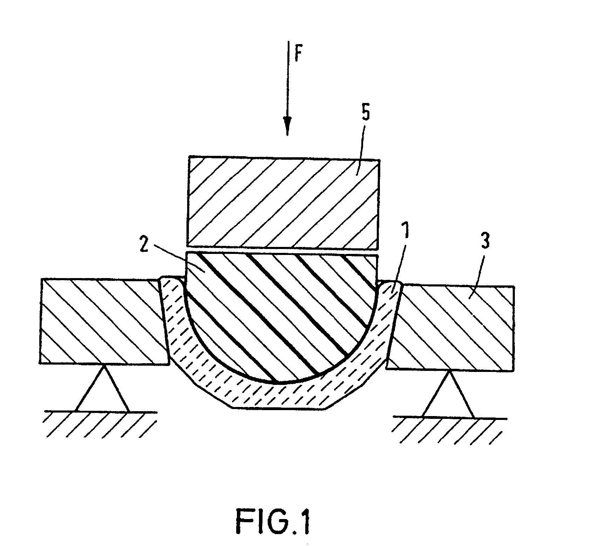

Fig. 1 zeigt eine Ausführungsform der Prüfvorrichtung nach dem erfindungsgemäßen Verfahren. In einer Haltevorrichtung 3 ist eine keramische Pfanne 1 für eine Hüftgelenkendoprothese so eingesetzt, daß sie sich nach der Prüfung leicht wieder entfernen läßt. Die untere Hälfte der Pfanne 1 ragt aus der Haltevorrichtung 3 heraus. Die Haltevorrichtung 3 ist hier als Platte ausgebildet. Zur Prüfung wird in die Pfanne 1 eine Halbkugel 2 aus einem verformbaren Material eingesetzt und über einen Stempel 5 mit einer Last beaufschlagt, die ein Vielfaches des maximalen physiologischen Lastfalls beträgt. Damit alle im physiologischen Lastfall unter Last stehenden Volumenelemente der Pfanne 1 belastet werden, füllt die Halbkugel 2 die Pfanne 1 vollständig aus. Bei einem Fehler im Volumen bzw. auf der Oberfläche der Pfanne 1 bricht diese. Es werden daher alle fehlerhaften Pfannen 1 zerstört.

Das verformbare Material der Halbkugel 2 besteht vorteilhafterweise aus einem Polymer oder einem Kunststoff, wie z. B. Polytetrafluorethylen, Polyurethan oder Silikonkautschuk oder aus Teflon.

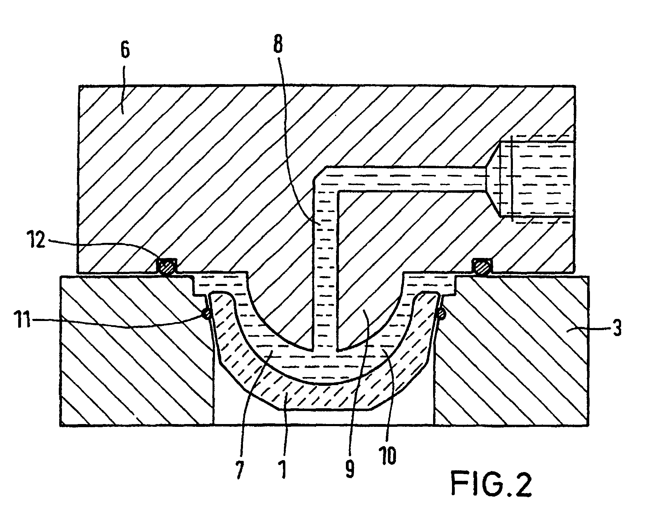

Fig. 2 zeigt eine bevorzugte Ausführungsform bei der die Innenseite der Pfanne 1 mit einer unter Druck stehenden Flüssigkeit 7 belastet wird. Die zu prüfende Pfanne 1 wird mit einem Dichtungselement 11 in die Haltevorrichtung 3 eingesetzt, wobei das Dichtungselement 11 die Pfanne vorzugsweise nur an ihrem konisch geformten oberen Ende abstützt. Auf die Haltevorrichtung 3 mit der eingesetzten Pfanne 1 ist ein Prüfstempel 6 aufgesetzt, der in einer bevorzugten Ausführungsform eine Ausbuchtung 9 aufweist, mit der er in die Pfanne 1 ragt, so daß nur ein Spalt 10 zwischen dem Prüfstempel 6 bzw. deren Ausbuchtung 9 und der Innenseite der Pfanne frei bleibt. Zwischen Prüfstempel 6 und Haltevorrichtung 3 ist ein Dichtungselement 12 angeordnet, das das innere Volumen der Pfanne 1 nach hin abdichtet.

Im Prüfstempel 6 und der Ausbuchtung 9 ist eine Zuführleitung 8 angeordnet, über die Flüssigkeit unter Druck in den Spalt 10 eingebracht werden kann.

Bei der Prüfung einer Pfanne 1 wird diese in die Haltevorrichtung 3 eingesetzt und der Prüfstempel 3 aufgesetzt. Anschließend wird über die Zuführleitung 8 Flüssigkeit unter Druck zugeführt, wodurch in der Pfanne eine Spannung entsteht, die um einen definierten Faktor höher ist, als die im physiologischen Lastfall auftretende Spannung. Pfannen mit als kritisch anzusehenden Defekten werden bei dieser Prüfung zerstört. Diejenigen Pfannen, die den Test bestanden haben, weisen eine Festigkeit auf, die höher ist als die durch den Überlasttest definierte Grenze.

## Patentansprüche

1. Verfahren zum Prüfen von keramischen Pfannen (1) für Hüftgelenkendoprothesen, mit einem im Oberschenkelknochen verankerbaren metallischen Schaft mit einem Zapfen und einem darauf aufgesetzten keramischen Kugelkopf, der in die keramische Pfanne (1) einsetzbar ist, die wiederum direkt oder über eine äußere Schale im Beckenknochen verankerbar ist, **dadurch gekennzeichnet,**
- **daß** die Innenseite der Pfanne (1) einer Last ausgesetzt wird,
- **daß** dabei alle im physiologischen Lastfall unter Last stehenden Volumenelemente der Pfanne (1) belastet werden und
- **daß** dabei in der Pfanne Spannungen erzeugt werden, die um einen definierten Faktor höher sind, als die im physiologischen Lastfall erzeugten Spannungen.

2. Verfahren nach Anspruch 1, **dadurch gekennzeichnet, daß** eine Halbkugel (2) aus einem verformbaren Material unter Last in die Pfanne (1) gedrückt wird.

3. Verfahren nach Anspruch 2, **dadurch gekennzeichnet, daß** das verformbare Material der Halbkugel (2) ein Polymer oder Kunststoff ist.

4. Verfahren nach Anspruch 1, **dadurch gekennzeichnet, daß** die Innenseite der Pfanne (1) mit einer unter Druck stehenden Flüssigkeit (7) belastet wird.

5. Verfahren nach Anspruch 4, **dadurch gekennzeichnet, daß** ein Prüfstempel (6) auf die Pfanne (1) aufgesetzt wird, der das Innere der Pfanne (1) nach außen mit einem Dichtungselement (12) abdichtet und eine Zuführleitung (8) für die Flüssigkeit (7) aufweist.

6. Verfahren nach Anspruch 5, **dadurch gekennzeichnet, daß** der Prüfstempel (6) mit einer Ausbuchtung (9) in die Pfanne (1) ragt, die nur einen Spalt (10) für die Flüssigkeit (7) freiläßt.

7. Verfahren nach einem der Ansprüche 1 bis 6, **dadurch gekennzeichnet, daß** die Pfanne (1) zur Prüfung in eine Haltevorrichtung (3) eingesetzt wird.

8. Verfahren nach Anspruch 7, **dadurch gekennzeichnet, daß** die Haltevorrichtung (3) die Pfanne (1) nur an ihrem konisch geformten oberen Ende mittels eines Dichtungselements (11) abstützt.

9. Verfahren nach einem der Ansprüche 4 bis 6, **dadurch gekennzeichnet, daß** die Flüssigkeit (7) eine für Hochdruck geeignete Flüssigkeit, wie Wasser oder Glycerin ist.

## Claims

1. Method for testing ceramic sockets (1) for hip-joint endoprostheses, having a metallic shaft, which can be anchored in the femur, with a pin and a ceramic ball head which is set thereon and can be inserted into the ceramic socket (1) which in turn can be anchored in the pelvic bone either directly or by way of an outer shell, **characterised**
- **in that** the inside of the socket (1) is subjected to a load,
- **in that** all the volume elements of the socket (1) which are under load in the case of a physiological load
are loaded thereby, and
- **in that** stresses which are higher by a defined factor than the stresses generated by a physiological load are thereby generated in the socket.

2. Method according to claim 1, **characterised in that** a hemisphere (2) that is made of a deformable material is pressed under load into the socket (1).

3. Method according to claim 2, **characterised in that** the deformable material of the hemisphere (2) is a polymer or plastics material.

4. Method according to claim 1, **characterised in that** the inside of the socket (1) is loaded with a liquid (7) that is under pressure.

5. Method according to claim 4, **characterised in that** a testing punch (6) is applied to the socket (1), which punch (6) seals the interior of the socket (1) from the exterior of the socket with a sealing element (12) and has a feed line (8) for the liquid (7).

6. Method according to claim 5, **characterised in that** the testing punch (6) projects into the socket (1) with a protruding portion (9) which leaves free only a gap (10) for the liquid (7).

7. Method according to one of claims 1 to 6, **characterised in that**, for testing purposes, the socket (1) is inserted into a holding arrangement (3).

8. Method according to claim 7, **characterised in that** the holding arrangement (3) supports the socket (1) only at its conically shaped upper end by means of a sealing element (11).

9. Method according to one of claims 4 to 6, **characterised in that** the liquid (7) is a liquid that is suitable for high pressure, such as water or glycerine.

## Revendications

1. Procédé de contrôle de cotyles prothétiques (1) céramiques pour endoprothèses de hanches, avec une tige métallique à ancrer dans le fémur, avec un axe et, montée sur celui-ci, une rotule céramique qui peut être placée dans le cotyle prothétique (1) céramique et à son tour est ancrée dans l'os du bassin directement ou par l'intermédiaire d'un insert extérieur, **caractérisé**
- **en ce qu'**on soumet la face intérieure du cotyle prothétique (1) céramique à une charge
- **en ce qu'**on charge tous les éléments de volume du cotyle prothétique (1) céramique qui sont soumis à une charge dans une situation de charge physiologique et
- **en ce qu'**on produit dans le cotyle prothétique des contraintes qui sont plus élevées d'un facteur donné que les contraintes produites dans la situation de charge physiologique.

2. Procédé selon la revendication 1, **caractérisé en ce qu'**on applique sous charge dans le cotyle prothétique (1) une demie sphère (2) en un matériau déformable.

3. Procédé selon la revendication 2, **caractérisé en ce que** le matériau de la demie sphère (2) est un polymère ou une matière plastique.

4. Procédé selon la revendication 1, **caractérisé en ce qu'**on charge la face intérieure du cotyle prothétique (1) avec un liquide (7) sous pression.

5. Procédé selon la revendication 4, **caractérisé en ce qu'**on place sur le cotyle prothétique (1) un tampon de contrôle (6) qui assure l'étanchéité de l'intérieur du cotyle prothétique (1) vis-à-vis de l'extérieur à l'aide d'un élément d'étanchéité (12) et présente une conduite d'entrée (8) pour le liquide (7).

6. Procédé selon la revendication 5, **caractérisé en ce que** le tampon de contrôle (6) fait saillie dans le cotyle prothétique (1) avec un renflement (9) qui ne laisse libre qu'une fente (10) pour le liquide (7).

7. Procédé selon une des revendications 1 à 6, **caractérisé en ce que** le cotyle prothétique (1), pour le contrôle, est placé dans un dispositif de support (3).

8. Procédé selon la revendication 7, **caractérisé en ce que** le dispositif de support (3) supporte le cotyle prothétique (1) à l'aide d'un élément d'étanchéité (11) uniquement au niveau de son extrémité supérieure de forme conique.

9. Procédé selon une des revendications 4 à 6, **caractérisé en ce que** le liquide (7) est un liquide adapté pour des pressions élevées, tel que de l'eau ou de la glycérine.
